# EUROPÄISCHE PATENTANMELDUNG

(11) **EP 3 682 917 A1**
(43) Veröffentlichungstag der Anmeldung: **22.07.2020**
(21) Anmeldenummer: 19151949.5
(22) Anmeldetag: 15.01.2019
(51) Int. Cl.: A61M 1/10

(54) **KÜHLUNG EINES ANTRIEBSSYSTEMS FÜR MEMBRANPUMPEN**

(71) Anmelder: Berlin Heart GmbH, 12247 Berlin (DE)
(72) Erfinder: Oakley, Matthew, 48231 Warendorf (DE); Schwiertz, Norbert, 15344 Strausberg (DE)
(74) Vertreter: Pfenning, Meinig & Partner mbB

(57) **Zusammenfassung**

Gegenstand der vorliegenden Erfindung ist eine Antriebseinheit (31) für eine Membranpumpe (3), wobei die Antriebseinheit einen Hohlkörper (33) und einen im ersten Hohlkörper entlang einer Achse des Hohlkörpers beweglich angeordneten Kolben (39) umfasst, wobei der Kolben den Hohlkörper in eine erste, mit der Membranpumpe verbindbare Kammer (41) und eine zweite, mit einem Gasreservoir koppelbare Kammer (43) teilt. Die zweite Kammer umfasst ein Einlassventil (61) und ein Auslassventil (65), so dass mittels des Einlassventils ein Gasstrom in die Kammer gesogen und mittels des Auslassventils aus der Kammer gedrückt wird.

## Beschreibung

Gegenstand der vorliegenden Erfindung ist eine Antriebseinheit, insbesondere für eine Membranpumpe, gemäß dem Oberbegriff des Anspruchs 1.

Membranpumpen kommen unter anderem bei extrakorporalen Herzunterstützungssystemen, sogenannten Ventricular Assist Devices (VADs), zum Einsatz. Um die Membranpumpen sehr genau steuern zu können, müssen zahlreiche Faktoren zusammenspielen. Unter anderem ist es wichtig, dass die Antriebseinheit die Membranpumpe zeit- und volumengenau ansteuert, so dass das in der Membranpumpe befindliche Blut in den Kreislauf eingepumpt beziehungsweise aus diesem ausgesogen werden kann.

Die Zuverlässigkeit der Antriebseinheit ist dabei von hoher Wichtigkeit, damit dem Patienten eine ausreichende Blutzufuhr garantiert ist. Das Antriebssystem soll dabei die aufgrund der Reibung beziehungsweise aufgrund der Drücke auftretende Wärme gut abführen können. Es hat sich gezeigt, dass insbesondere die vorzunehmenden Wartungen ansteigen, wenn die Wärme nicht zuverlässig aus der Antriebseinheit des Antriebssystems abtransportiert werden kann. Eine ungleichmäßige Verteilung der Wärme innerhalb des Antriebssystems in einer Antriebseinheit führt zu lokalen Hotspots, welche beispielsweise Undichtigkeiten bewirken, die wiederum eine ungleichmäßigere Druckluftzufuhr zur Membranpumpe zur Folge haben.

Es ist daher die Aufgabe der vorliegenden Erfindung, die Kühlung beziehungsweise Wärmeverteilung innerhalb eines Antriebssystems für Membranpumpen zur Herzunterstützung zu verbessern. Die Aufgabe wird unter anderem durch eine Antriebseinheit für eine Membranpumpe gemäß Anspruch 1 gelöst.

Die Antriebseinheit für eine Membranpumpe umfasst einen ersten Hohlkörper und einen im ersten Hohlkörper entlang einer Achse des Hohlkörpers beweglich angeordneten Kolben. Der Kolben unterteilt den Hohlkörper in einer erste, mit der Membranpumpe verbundene Kammer und eine zweite, mit einem Gasreservoir koppelbare Kammer. Bei dem Hohlkörper kann es sich beispielsweise um einen Zylinder handeln, in dem ein runder Kolben läuft. Durch die in der ersten Kammer vorhandene Luft (oder ein anderes Gas oder Fluid) kann die Membran der Membranpumpe angehoben beziehungsweise entspannt werden und hierdurch ein Unterdruck beziehungsweise Überdruck in der zweiten Kammer der Membranpumpe erzeugt werden, so dass Blut ausgestoßen beziehungsweise eingesogen werden kann.

Aufgrund der Kompression des Gases bzw. der Luft in der ersten Kammer wird der Hohlkörper erwärmt. Diese Wärme kann zum einen über den Hohlkörper selbst und zum anderen über das Gas, das sich in der zweiten Kammer der Antriebseinheit befindet, aus der Antriebseinheit selbst ausgeführt werden.

Die zweite Kammer ist mit der Umwelt über ein Einlassventil und ein Auslassventil verbunden. Dabei wird mittels des Einlassventils ein Gasstrom, in der Regel Umgebungsluft, aus der äußeren Umgebung in die Kammer gesogen und über das Auslassventil aus der Kammer gedrückt. Mithilfe des Einlass- und das Auslassventils ist es möglich, einen gerichteten Luftstrom in der zweiten Kammer der Antriebseinheit zu erzeugen. Hierdurch wird gewährleistet, dass durch das Einlassventil ausschließlich Luft von außerhalb der Antriebseinheit in die zweite Kammer gesogen wird und diese Luft vorzugsweise vollständig über das Auslassventil an die Außenumgebung abgegeben wird. In zahlreichen Ausführungsformen sind das Einlassventil und das Auslassventil voneinander getrennt ausgebildet. Hierdurch wird also gewährleistet, dass die Luft, die oftmals, wenn sie von außen eingesogen wird, kälter ist als die Luft in der zweiten Kammer, sich in der zweiten Kammer - erwärmt und erwärmte Luft vollständig über das Auslassventil ausgestoßen wird. Mithilfe des Einlass- und Auslassventils ist es also möglich, einen gerichteten Luftstrom durch die zweite Kammer zu erzeugen. Auf diese Weise kann die Temperatur innerhalb der Antriebseinheit geringer gehalten und gleichmäßig verteilt, d. h. ohne lokale Hotspots, eingestellt werden.

Weitere Aspekte der Erfindung ergeben sich aus den untergeordneten Ansprüchen.

Das Einlass- und/oder das Auslassventil können vorzugsweise als Rückschlagventil ausgebildet sein. Rückschlagventile können beispielsweise einfach aufgebaut sein, zum Beispiel mechanische Rückschlagventile. Die Rückschlagventile ermöglichen einen gerichteten Luftstrom und benötigen keinerlei Schaltungselektronik. Ferner können die Rückschlagventile auch so ausgewählt werden, dass die die Ventile öffnende Kraft an die Betriebsparameter der Antriebseinheit angepasst ist. Die Rückschlagventile können beispielsweise federbelastet sein oder elektronisch angesteuerte Rückschlagventile sein. In anderen Variationen kann eines der Ventile auch als Absperrventil, das elektronisch reguliert wird, ausgebildet sein.

Ergänzend oder optional kann die Antriebseinheit eine Filtereinheit aufweisen, wobei das Einlassventil vorzugsweise die Filtereinheit und die zweite Kammer fluide miteinander koppelt. Die Filtereinheit stellt sicher, dass Schmutz und Schwebepartikel nicht in die zweite Kammer gelangen können. Hierdurch werden die Wartungszyklen für die Antriebseinheit verlängert. Bei der Filtereinheit können beispielsweise ein textiler Filter, ein Keramikfilter oder sonstige im Stand der Technik bekannte Filter zur Luftfilterung verwendet werden.

Alternativ oder zusätzlich handelt es sich bei der Filtereinheit um einen austauschbaren Filter, wobei der Filter vorzugsweise zwischen einer Filterschale und einem Filterdeckel gehalten ist. Eine austauschbare Filtereinheit hat den Vorteil, dass in regelmäßigen Wartungszyklen die Filtereinheit getauscht werden kann und so eine Verstopfung des Filters vermieden wird.

Alternativ oder ergänzend kann die Antriebseinheit so ausgebildet sein, dass Verbindungselemente, die das Einlass- beziehungsweise Auslassventil mit der zweiten Kammer verbinden, einen voneinander unterschiedlichen Querschnitt aufweisen. So kann beispielsweise die mit dem Einlassventil verbundene Röhre einen geringeren Querschnitt aufweisen als die mit dem Auslassventil verbundene Röhre. Hierdurch wird der eingesogene Luftstrom mit höherer Geschwindigkeit eingesogen, so dass die zweite Kammer starke Verwirbelungen aufweist und die eingesogene Luft gleichmäßig über die erste Kammer verteilt wird. Dadurch, dass die Querschnittsfläche des mit dem Auslassventil verbundenen Verbindungselements größer ist, wird die Luft beziehungsweise das eingesogene Gas beim Ausstoßen nicht zusätzlich erwärmt, sondern lediglich sanft durch den Auslass beziehungsweise das Auslassventil gedrückt.

Alternativ oder ergänzend umfasst die Antriebseinheit ferner mindestens einen Kühlkörper, wobei das Auslassventil den Kühlkörper mit der zweiten Kammer fluide koppelt. Die Kühlkörper sind dabei so angeordnet, dass sie in unmittelbarer Nähe eines Motors angebracht sind, der den im Hohlkörper angeordneten Kolben bewegt. Die durch den Motor erzeugte Abwärme wird dann über die Kühlkörper gleich an die Umgebung abgeführt. Hierbei ist es vorteilhaft, wenn der Motor mit dem mindestens einen Kühlkörper thermisch gekoppelt ist.

Neben dem gerichteten Luftstrom kann der Wärmeeintrag in die Antriebseinheit ferner reduziert werden, wenn sich zwischen den Innenwand des Hohlkörpers und dem Kolben eine Spaltdichtung befindet. Diese Spaltdichtung bildet eine zwischen der ersten und der zweiten Kammer aufgebaute Barriere. Die Spaltdichtung kann beispielsweise mindestens einen O-Ring umfassen, der vorzugsweise in mindestens einer Nut des Kolbens geführt ist. Bei dem O-Ring kann es sich beispielsweise um einen O-Ring aus Viton handeln.

Eine weitere Maßnahme zur Regulierung der Wärme ist es, die Innenseite des Hohlkörpers mit einer thermischen Beschichtung zu bedecken. Als thermische Beschichtungen kommen hier beispielsweise diamantenartiges Karbon (diamondlike carbon; DLC) oder PTFE-Beschichtungen in Frage.

Alternativ oder zusätzlich kann die Außenwand des Hohlkörpers Kühlrippen aufweisen, die die in den Hohlkörper eingebrachte Wärme ebenfalls besser nach außen abführen sollen.

Weitere Ausführungsformen werden in den nachstehenden Ausführungsbeispielen erläutert. Dabei können die vorgenannten Merkmale jeweils einzeln und getrennt voneinander in den Ausführungsbeispielen realisiert werden, auch wenn die Ausführungsbeispiele selbst mehrere der einzelnen Merkmale umfassen.

Es zeigt
- Fig. 1: eine schematische Ansicht über ein VAD-System mit zwei Membranpumpen und einem Antriebssystem mit einer Steuerungseinheit und zwei Antriebseinheiten;
- Fig. 2: einen schematischen Längsschnitt durch eine Antriebseinheit;
- Fig. 3: eine räumliche Darstellung einer Antriebseinheit;
- Fig. 4: einen Querschnitt durch die Antriebseinheit der Figur 3;
- Fig. 5: einen weiteren Querschnitt durch die Antriebseinheit der Fig. 3;
- Fig. 6: einen Längsschnitt durch die Antriebseinheit der Fig. 3; und
- Fign. 7A-D: eine schematische Darstellung eines Einsaug- und Ausstoßvorgangs mittels einer hier beschriebenen Antriebseinheit.

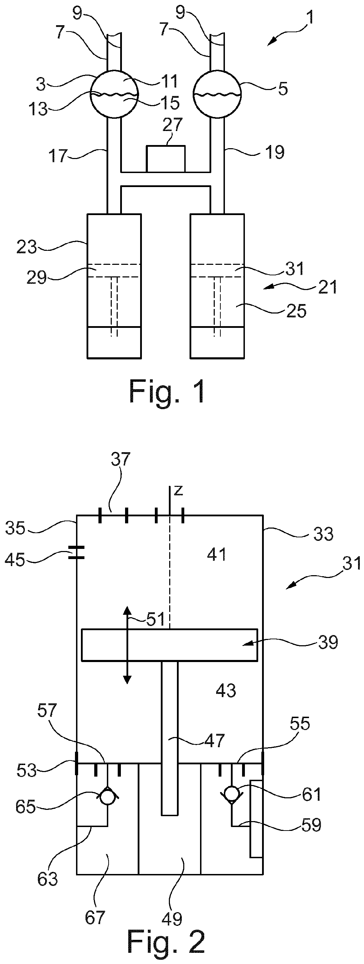

Figur 1 zeigt schematisch ein extrakorporales VAD-System mit Antriebseinheit, wie es beispielsweise kommerziell von Berlin Heart als ®EXCOR mit der Antriebseinheit IKUS erhältlich ist. Das VAD-System 1 umfasst eine erste Membranpumpe 3 und eine zweite Membranpumpe 5, die mittels Leitungen 7 und 9 mit einer Kammer des Herzens, beispielsweise dem linken Ventrikel, verbunden sind. Dabei wird die Pumpe so konfiguriert, dass beispielsweise durch die Schlauchleitung das Blut in die erste Kammer 11 der Membranpumpe gesogen wird und über die zweite Leitung 9 aus der Kammer 11 ins Herz ausgestoßen wird.

Innerhalb der Membranpumpe ist eine Membran 13 angeordnet, die die erste Kammer 11, die mit Blut befüllt werden kann, von der Luftkammer 15 trennt. Die Luftkammer 15 ist über einen weiteren Schlauch 17 mit dem Antriebssystem verbunden. Das Antriebssystem 21 umfasst eine erste Antriebseinheit 23 und eine zweite Antriebseinheit 25, die jeweils mit einer der Membranpumpen 3 und 5 über eine der Luftleitungen 17 und 19 verbunden ist. Die Antriebseinheiten 23 und 25 werden mittels einer Steuereinheit 27 angesteuert, so dass der in den Antriebseinheiten 23 und 25 angeordnete Kolben 29 beziehungsweise 31 die Kammer 15 beziehungsweise die entsprechende Kammer der Membranpumpe 5 mit Gas befüllt beziehungsweise das Gas absaugt.

Anhand der Figur 2 soll die Antriebseinheit 23 näher erläutert werden. Die in Figur 2 dargestellte Antriebseinheit 31 umfasst einen Hohlkörper 33, der im vorliegenden Beispiel als Zylinder mit Zylinderachse z ausgebildet ist. Der Zylinder ist an seinem oberen Ende mittels eines Deckels 35 luftdicht verschlossen und weist eine Öffnung 37 auf, die mit einem Luft- oder Gasschlauch, wie beispielsweise dem Schlauch 17 oder 19, verbunden werden kann. Alternativ zu einer reinen Öffnung kann auch ein Port zum direkten Anschließen des Schlauchs vorhanden sein.

Innerhalb des Hohlkörpers 33 ist ein Kolben 39 angeordnet, der den Innenraum des Hohlkörpers 33 in eine erste Kammer 41 und eine zweite Kammer 43 teilt. Die erste Kammer 41 ist so ausgelegt, dass sie fluide mit den Membranpumpen oder einer der Membranpumpen verbunden werden kann. Die zweite Kammer 43 ist so ausgebildet, dass sie mit Gas oder Luft befüllt werden kann und dieses Gas beziehungsweise die Luft ausgestoßen werden kann. Sollte es aus irgendeinem Grund beispielsweise zu Leckagen in der ersten Kammer 41 kommen, kann diese mittels eines weiteren Ports 45 zusätzliche Luft einsaugen. Der Port kann dabei beispielsweise mit einer Filtereinheit verbunden sein.

Der Kolben 39 ist mit einer Spindel 47 verbunden, die mittels einer Antriebseinheit 49 in Form eines elektrischen Motors rotiert wird. Hierdurch kann ein Kolbenhub 51 erzeugt werden, so dass das Volumen der ersten Kammer 41 verringert und das Volumen der zweiten Kammer 43 entsprechend vergrößert wird oder umgekehrt.

Die untere Kammer 43 ist mittels einer unteren Abdeckung 53 abgesperrt. Die Spindel 47 wird mittels Dichtungen durch die als Platte ausgeführte Abdeckung 53 geführt. Ferner weist die Platte einen Einlass 55 und einen Auslass 57 auf. Der Einlass ist mit einer Leitung 59 mit der Außenwelt verbunden. In dieser Leitung ist ein Einlassventil 61 angeordnet, das im vorliegenden Beispiel als Rückschlagventil so konfiguriert ist, dass Luft durch den Einlass 55 nicht in die Außenwelt austreten kann. Analog hierzu ist der Auslass 57 mittels einer Leitung 63 mit der Außenwelt verbunden, wobei in der Leitung 63 ein Auslassventil 65 vorhanden ist, das in Auslassrichtung Luft austreten lässt und in Einsaugrichtung sperrt. Die Auslassleitung 63 befindet sich in einem Kühlkörper 67, der thermisch mit dem Motor 49 gekoppelt ist, indem auf den Motor eine Wärmeleitpaste aufgetragen wurde, die mit dem Kühlkörper verklebt ist.

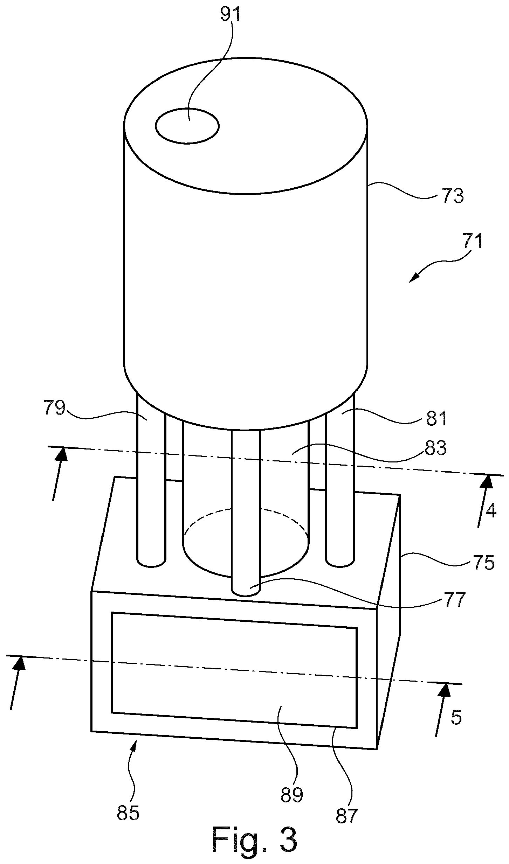

In Figur 3 wird in räumlicher Darstellung nochmals eine Antriebseinheit gezeigt. Die Antriebseinheit 71 umfasst einen zylinderförmigen Hohlkörper 73 sowie einen unterhalb des Hohlkörpers 73 angeordneten Antrieb innerhalb des Ansaug- beziehungsweise Ausstoßblock 75, der mit dem Hohlkörper 73 mittels der Einlassleitung 77 und den Auslassleitungen 79 und 81 gekoppelt ist. In der Einlassleitung 77 ist ein Einlassventil, in den Auslassleitungen 79 und 81 jeweils ein Auslassventil angeordnet. Eine in Figur 3 nicht dargestellte Spindel verläuft durch den Block 83, der den Motor mit dem Kolben koppelt. Der Ansaugblock75 umfasst neben einem in dieser Darstellung nicht gezeigten Motor eine Filtereinheit 85, die einen Filterrahmen 87, einen dahinter liegenden austauschbaren Filter 89 aus Textil und eine durch den austauschbaren Filter 89 ausgefüllte Filterschale 101 umfasst. Am oberen Ende des Hohlkörpers 73 befindet sich ein Port 91, über den eine Kammer des Hohlkörpers mit einer Membranpumpe verbunden werden kann. Der hier dargestellte Hohlkörper ist aus einem Metall gefertigt und kann in einer anderen Ausführungsform Kühlrippen aufweisen, die den Hohlkörper außen umlaufen. Der Antriebsblock 75 kann auch aus mehreren Komponenten gebildet werden und aus Metall bestehen, wie beispielsweise aus gefrästem Aluminium oder Titan.

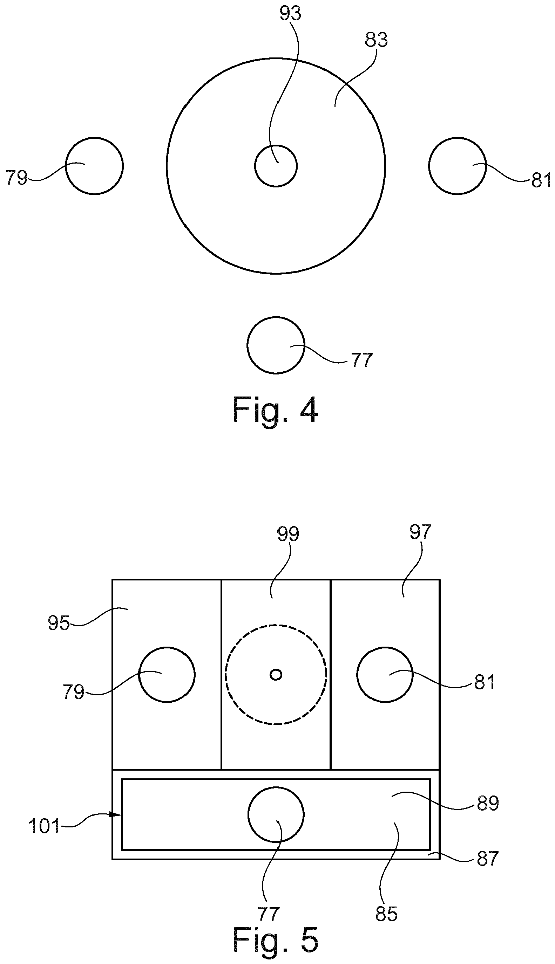

In Figur 4 ist ein Querschnitt durch die Auslassleitungen 79 und 81, die Einlassleitungen 77 sowie den Block 83 gezeigt, in dem die Spindel 93 verläuft. Es ist deutlich erkennbar, dass der Querschnitt des Einlasses 77 kleiner ist als der Querschnitt der kombinierten Auslässe 79 und 81. Auf diese Weise wird der Luft- beziehungsweise Gasstrom beim Ansaugen beschleunigt und in die Kammer geführt und sorgt so für eine zusätzliche Durchmischung der in der zweiten Kammer des Hohlkörpers vorhandenen Luft. Beim anschließenden Ausstoßen der Luft steht ein größerer Querschnitt zur Verfügung, so dass die Luft nur in geringem Maße zusätzlich erwärmt wird.

In Figur 5 ist ein weiterer Querschnitt durch die Antriebseinheit der Figur 3 gezeigt. Wiederum sind die Auslassleitungen 79 und 81 erkennbar, die durch Kühlkörper 95 und 97 verlaufen. Die Kühlkörper grenzen dabei direkt an die Antriebseinheit 99 an, mittels welcher die Spindel 93 angetrieben wird, um den Kolben auf und ab zu bewegen. Ferner ist die Filtereinheit 85 erkennbar, mit der die Einlassleitung 77 fluide gekoppelt ist. In der Darstellung der Figur 5 sind auch der Filterrahmen 87, die austauschbare Filtereinheit 89 und die Filterschale 101 erkennbar, in der der austauschbare Filter 89 anliegt. Der Filterdeckel 87 ist dabei mittels Schrauben mit der Filterschale 101 gekoppelt. Anhand der Kühlkörper, die beispielsweise aus gefrästem Metall bestehen können, kann die Wärme, die im Antrieb 99 erzeugt wird, besser an die Außenwelt abgegeben werden.

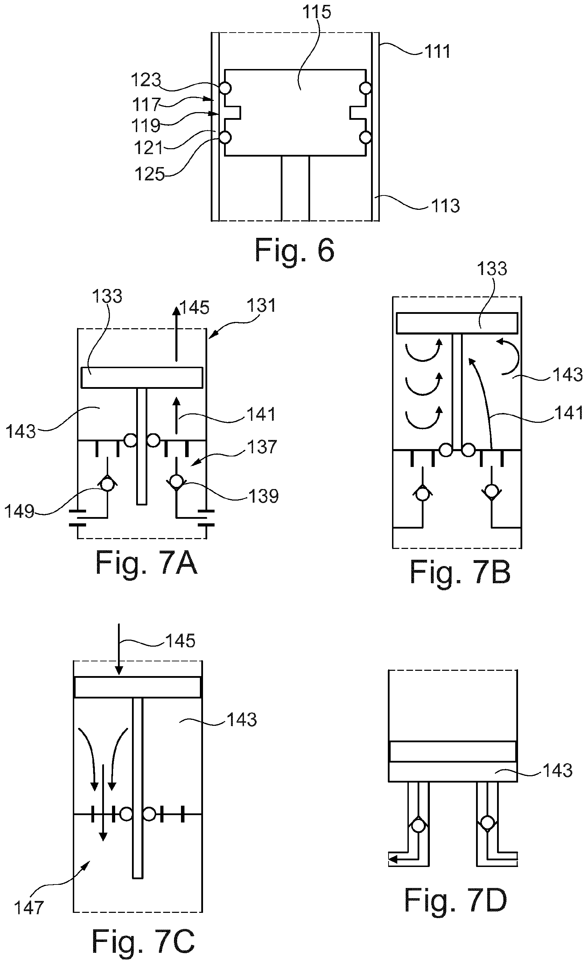

In Figur 6 sind weitere Maßnahmen zur Ableitung der Wärme aus der Antriebseinheit gezeigt. Der Hohlkörper 111 weist auf seiner Innenseite eine Beschichtung 113 aus diamantartigem Karbon auf, die sich durch besondere Glattheit und damit durch verminderte Reibung auszeichnet. Der Kolben 115 weist drei Nuten 117, 119, 121 auf, die den Kolben kreisrund umlaufen. In den Nuten 117 und 121 ist jeweils ein Ring aus Viton angeordnet, der einen Abschluss zwischen der Außenwand des Kolbens und der Innenwand des Hohlkörpers 111 bildet. In der Nut 119 befindet sich ein Reservoir für einen vernetzten Fettfilm, mit dem die Reibung zwischen dem O-Ring 123 beziehungsweise 125 und der Innenwand des Hohlkörpers 111 reduziert werden soll.

Anhand der Abfolge der Figuren 7A-D soll noch kurz auf den Einsaugvorgang und den Ausstoßvorgang der zweiten Kammer der Antriebseinheit eingegangen werden. In den Figuren 7A-D ist dabei jeweils nur ein Ausschnitt der Antriebseinheit dargestellt, und insbesondere lediglich der Kolben, die zweite Kammer sowie ein schematischer Einlass und ein schematischer Auslass.

Bezüglich der Antriebseinheit 131 ist in Figur 7A dargestellt, dass sich der Kolben 133 in die Richtung 135 nach oben bewegt. Hierdurch wird durch den Einlass 137, der ein Rückschlagventil 139 aufweist, Luft 141 in die zweite Kammer 143 gesogen. Mit zunehmendem Hub des Kolbens 133 strömt Luft 141 in die zweite Kammer 143 ein und sorgt für eine gute Durchmischung und Verteilung der eingesogenen Luft über die gesamte Kammer hinweg (siehe Figur 7B).

In Figur 7B hat die zweite Kammer ihr maximales Volumen. In der anschließenden Absenkbewegung 145 wie in Figur 7C dargestellt kann die in der zweiten Kammer 143 vorhandene Luft lediglich durch den Auslass 147 und das im Auslass angeordneten Rückschlagventil 149 entweichen. Hierdurch wird die durch die Luft aufgenommene Wärme der Außenwelt zugeführt und Wärme aus der Antriebseinheit heraustransportiert. Wenn die zweite Kammer ihr minimales Volumen erreicht hat (siehe Figur 7D), beginnt der Zyklus von neuem, und es wird neue kühle und gefilterte Luft durch den Einlass 141 in die Kammer gesogen.

Weitere Ausführungsbeispiele ergeben sich für den Fachmann in naheliegender Weise.

## Patentansprüche

1. Antriebseinheit (31) für eine Membranpumpe (35), mit einem ersten Hohlkörper (37) und einem im ersten Hohlkörper entlang einer Achse (z) des Hohlkörpers beweglich angeordneten Kolben (39), wobei der Kolben den Hohlkörper in eine erste, mit der Membranpumpe verbindbare Kammer (41) und eine zweite, mit einem Gasreservoir koppelbare Kammer (43) teilt,
wobei
die zweite Kammer mit einem Einlassventil (61) und einem Auslassventil (65) gekoppelt ist, so dass mittels des Einlassventils ein Gasstrom in die Kammer gesogen wird und mittels des Auslassventils aus der Kammer gedrückt wird.

2. Antriebseinheit nach Anspruch 1, wobei das Einlassventil und/oder das Auslassventil ein Rückschlagventil ist.

3. Antriebseinheit nach einem der Ansprüche, wobei eine Filtereinheit (85) vorhanden ist, und das Einlassventil vorzugsweise die Filtereinheit und die zweite Kammer fluide koppelt.

4. Antriebseinheit nach Anspruch 3, wobei die Filtereinheit einen austauschbaren Filter (89) aufweist, und der Filter vorzugsweise zwischen einer Filterschale (101) und einem Filterdeckel (87) gehalten ist.

5. Antriebseinheit nach einem der vorhergehenden Ansprüche, wobei mindestens ein Kühlkörper (95, 97) vorhanden ist, und das Auslassventil den Kühlkörper mit der zweiten Kammer fluide koppelt.

6. Antriebseinheit nach einem der vorhergehenden Ansprüche, wobei ein Einlass (55) der zweiten Kammer mit dem Einlassventil (61) gekoppelt und ein Auslass (57) der zweiten Kammer mit dem Auslassventil (65) gekoppelt ist.

7. Antriebseinheit nach einem der vorhergehenden Ansprüche, wobei ein den Kolben antreibender Motor (99) unterhalb der zweiten Kammer angeordnet ist.

8. Antriebseinheit nach den Ansprüchen 5 und 7, wobei der Motor mit dem mindestens einen Kühlkörper thermisch gekoppelt ist.

9. Antriebseinheit nach einem der vorhergehenden Ansprüche, wobei zwischen einer Innenwand des Hohlkörpers und dem Kolben eine Spaltdichtung angeordnet ist.

10. Antriebseinheit nach Anspruch 9, wobei die Spaltdichtung mindestens einen O-Ring (123, 125) umfasst, der vorzugsweise in mindestens einer Nut des Kolbens geführt ist.

11. Antriebseinheit nach einem der vorhergehenden Ansprüche, wobei der Hohlkörper eine thermische Beschichtung (113) aufweist.
